# EUROPEAN PATENT SPECIFICATION

(11) **EP 4 178 542 B1**
(45) Date of publication and mention of the grant of the patent: **04.12.2024**
(21) Application number: 21742454.8
(22) Date of filing: 07.07.2021
(51) Int. Cl.: A61K 9/10, A61K 9/06, A61K 9/00, A61K 31/519, A61K 47/02

(54) **SILICA HYDROGEL COMPOSITE AND ITS USE**
SILICA-HYDROGEL-VERBUNDSTOFF UND DESSEN VERWENDUNG
COMPOSITE D'HYDROGEL DE SILICE ET SON UTILISATION

(30) Priority: 07.07.2020 FI 20205732
(43) Date of publication of application: 17.05.2023
(73) Proprietor: DelSiTech Oy, 20520 Turku (FI)
(72) Inventor: VAAHTIO, Minna, 20520 Turku (FI); KAIMAINEN, Mika, 20520 Turku (FI); FORSBACK, Ari-Pekka, 20520 Turku (FI); JOKINEN, Mika, 20520 Turku (FI); LEINO, Lasse, 20520 Turku (FI)
(74) Representative: Laine IP Oy
(86) International application number: PCT/FI2021/050531
(87) International publication number: WO 2022/008804

(56) References cited:
- WO-A1-2008/104635
- WO-A1-2017/068245
- WO-A1-2017/072415
- WO-A1-2020/096210

## Description

### FIELD OF INVENTION

This invention relates to a silica hydrogel composite. More specifically this invention relates to a hydrogel composite comprising at least one active pharmaceutical ingredient as solid particles. The hydrogel composite is feasible for controlled and sustained delivery of active pharmaceutical ingredients.

### BACKGROUND OF THE INVENTION

Low solubility of active pharmaceutical ingredients (API) is a general challenge in the development of new drugs. One aspect is related to aqueous solubility of API to achieve desired concentration in systemic circulation or different body fluids, and good absorption and permeability. Another aspect is related to formulation and development of different dosage forms for controlled release, e.g., to solubility of API in water or in other solvents/liquids, which are used in preparation of different kind of medical biomaterials, such as silica or poly(lactic-co-glycolic acid) PLGA, which are used as matrix materials in controlled drug delivery. API is often incorporated, added, encapsulated or embedded into biomaterials that are used as matrix materials or API is added into process liquids of matrix materials in soluble form to ensure homogeneous distribution throughout the system and in final matrix material.

Conventionally for development of different formulations, matrix materials and dosage forms for controlled release, API is dissolved in water or in other solvents/liquids, such as ethanol, which are then used in preparation of different kind of matrix materials, such as silica. For example, when API is encapsulated in dosage forms that are based on sol-gel derived silica, it is preferable that API can be dissolved in water, in alcohol (e.g., in ethanol when using alkoxides, such as tetraethyl orthosilicate (TEOS) as a precursor for silica) or in water-alcohol mixtures, because the dissolved molecule form guarantees homogeneous distribution of API in the reaction liquid (e.g., in silica sol), which also increases possibility for homogeneous distribution of API in the final matrix material, e.g., as API becomes encapsulated in silica microparticles prepared by spray-drying of a silica sol comprising dissolved API. The use of solvents, however, must also match with the development of properties of the matrix material during the preparation. For example, if API does not dissolve in water, but API dissolves in high enough concentration in ethanol, it may occur that the pH, where this dissolution occurs is not suitable for development of silica species or silica microparticle properties during the processing, and a proper controlled release matrix cannot be prepared.

When API is dissolved in processing liquids of a matrix material, it is in a molecular form and homogeneously distributed as molecules in a processing liquid for matrix materials and when a matrix materials is further processed to or turning into a final dosage form to be used as a controlled delivery device, such as solid implants, solid particles, a hydrogel, or some other material or dosage form, where the solid phase dominates the matrix materials properties, the API may be in a solid phase, but the size of solid phase is small (because it is formed of molecular species of the active pharmaceutical ingredient homogeneously distributed in the system), or the API can still be partly dissolved in the molecular form (e.g., in the liquid phase of a hydrogel).

Different kind of gels, such as hydrogels on the hand are often relative loose structures due to typically small solid content. The low solid content in hydrogels is possible because the solid phase of hydrogels is commonly composed of crosslinked polymeric networks or other polymeric species, such as aggregated nanoparticles in the case of sol-gel derived silica. Due to molecular or nanoscale size of the polymer network backbone or nanoparticle aggregates, even low solid content is enough to reach distribution of the solid phase throughout the whole structure of a hydrogel. It means in practice that hydrogels are porous structures, in which the pores are filled with an aqueous solution. The controlled release properties depend on the type of hydrogel and the final solid content of hydrogels. Some hydrogels work as controlled release matrix due to suitable pore size, or due to swelling of pores in body fluid conditions. Some hydrogels, such as silica hydrogels, release the encapsulated or embedded API mainly due to slow dissolution of the solid phase of the hydrogel. Although the typical low solid content in hydrogels is challenging from the viewpoint of controlled release, it is beneficial from another viewpoint, i.e., they can be used in minimally invasive, thin-needle injections from syringes.

Jokinen et al. (WO 2014/207304) and Leino et al. (WO2017/068245) disclose silica hydrogel composites. API is first encapsulated or embedded in silica microparticles during the spray-drying, after which these API-containing silica microparticles are further embedded in a loose silica hydrogel.

Liu et al. (US6303290) disclose a process for the encapsulation of biologically important proteins into transparent, porous silica matrices by an alcohol-free, aqueous, colloidal sol-gel process. In particular, Liu et al. states that contact with alcohol is completely eliminated throughout the manufacturing process of the porous silica matrices encapsulating a biopolymer, thereby avoiding the alcohol-caused denaturalization of many biopolymers (caused by chain unfolding or molecule aggregation), typically seen in the conventional encapsulation methods.

Niskanen et al. (WO 2017/072415) discloses compositions for the sustained release of NSAIDs.

### OBJECT AND SUMMARY OF THE INVENTION

An object of this invention is to minimize or possibly even eliminate the disadvantages existing in the prior art.

One object of the present invention is to provide a silica hydrogel composite comprising active pharmaceutical ingredients as solid particles.

One specific object of the present invention is to provide a silica hydrogel composite comprising solid particles of Anagrelide or its pharmaceutically acceptable salts for controlled release.

A further object of the present invention is to provide a silica hydrogel composite comprising at least one active pharmaceutical ingredient for medical use in parenteral administration, e.g., by thin needle injection.

These objects are attained with the invention having the characteristics presented below in the characterizing parts of the independent claims. Some preferred embodiments of the invention are presented in the dependent claims.

The present invention provides a silica hydrogel composite comprising at least one active pharmaceutical ingredient, wherein the silica hydrogel composite is obtainable by mixing
a. non-active silica particles having a diameter of ≤ 100 µm,
b. a silica sol, having a solid content in the silica sol less than 3 weight-%, and
c. solid particles of at least one active pharmaceutical ingredient (API), the particles having preferably a diameter of ≤ 300 µm,
wherein the silica hydrogel composite comprises up to 75 weight-% of said non-active silica particles and wherein the silica hydrogel composite is non-flowing and structurally stable when stored at rest and shear-thinning when shear stress is applied by injection.

### BRIEF DESCRIPTION OF THE DRAWINGS

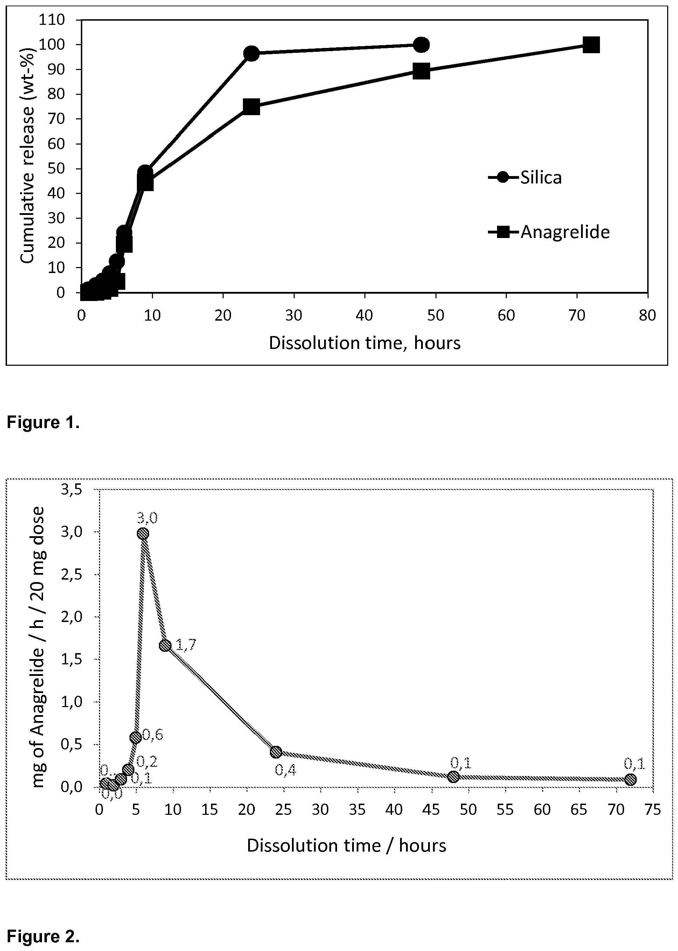

Figures 1 illustrates cumulative in vitro in sink silica dissolution rate and release rate of Anagrelide from silica hydrogel composite depot formulation #29HG (comprising silica hydrogel of R300, micronized API powder and non-active silica particles, pH 5.8) in 50 mM TRIS buffer with 0.5 % SDS (pH 7.4 at 37 °C). Mean values of triplicate analyses given at each time point..
Figure 2 illustrates cumulative in vitro in sink release rate profile (mg/hour) of Anagrelide from silica hydrogel composite with a dose of 20 mg of Anagrelide HCl. Silica hydrogel depot formulation #29HG in 50 mM TRIS buffer with 0.5 % SDS (pH 7.4 at 37 °C). Mean values of triplicate analyses given at each time point.

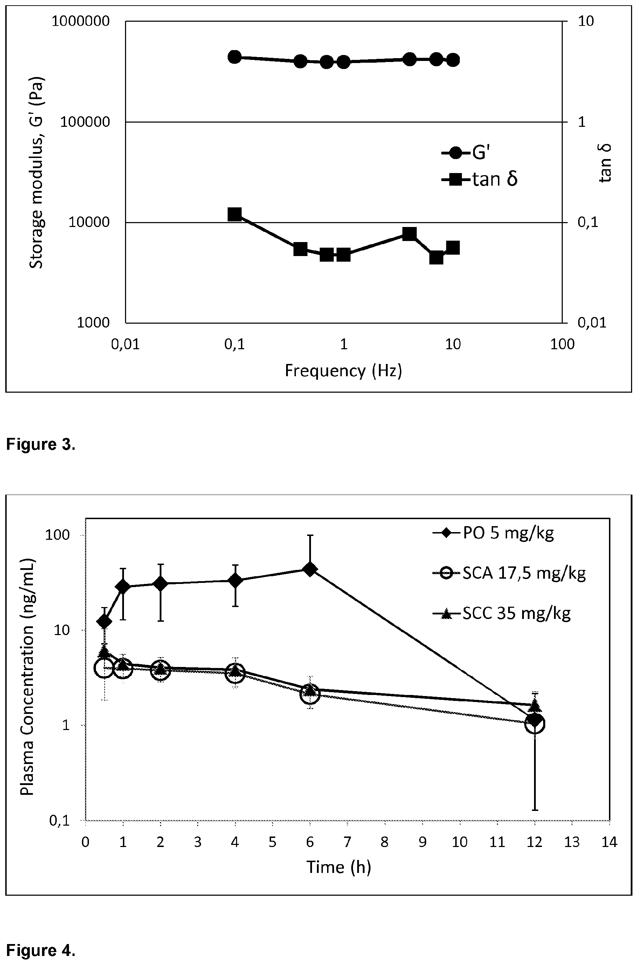

Figure 3 illustrates the rheological properties, i.e., stability at rest (as in a syringe) and shear-thinning under shear (as in injection from a syringe) for the silica hydrogel composite comprising Anagrelide. Storage modulus (G') and tan δ (loss factor=G"/G')) values for the silica hydrogel composite depot formulation #29HG (comprising silica hydrogel of R300, micronized API (Anagrelide HCl) powder and non-active silica particles, pH 5.8).
Figure 4 illustrates mean plasma concentrations of Anagrelide in a pharmacokinetic in vivo experiment for 12 hours after oral administration and subcutaneous injection of silica hydrogel composite comprising 2 different doses of Anagrelide HCl. Mean (average for 5 animals) plasma concentration of Anagrelide HCl as a function of time for 12 h experiment after oral administration (PO) of Anagrelide HCl and subcutaneous injections (SC) of silica hydrogel composite #29HG (depot) comprising Anagrelide HCl (17.5 and 35 mg/kg) into male SD rats.

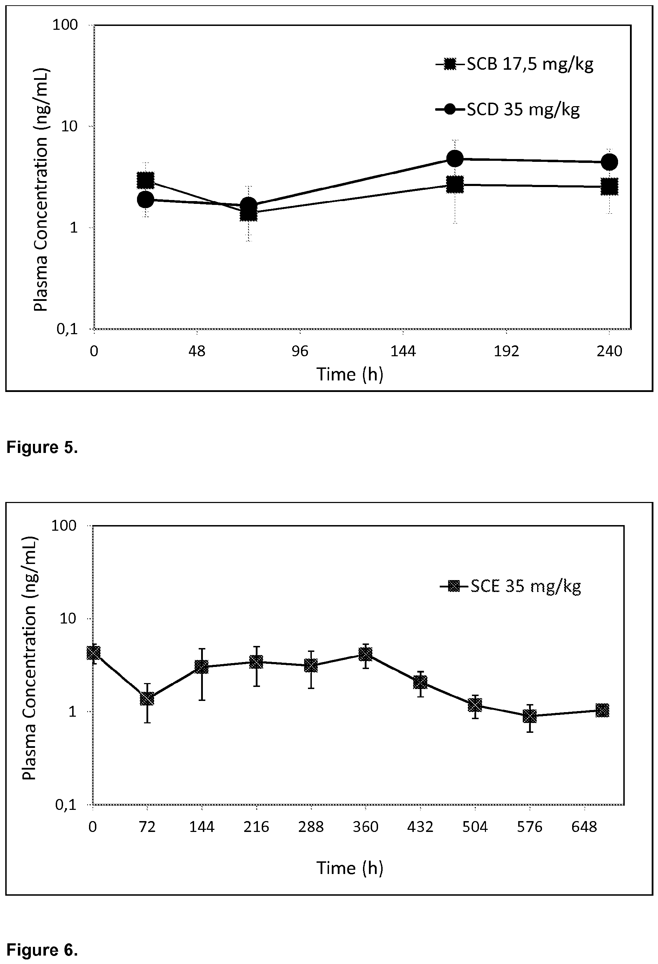

Figure 5 illustrates mean plasma concentrations of Anagrelide in a pharmacokinetic in vivo experiment for 10 days after subcutaneous injection of silica hydrogel composite comprising 2 different doses of Anagrelide HCl. Mean (average for 5 animals) plasma concentration of Anagrelide HCl as a function of time for 10 d experiment after subcutaneous injections (SC) of silica hydrogel composite #29HG (depot) comprising Anagrelide HCl (17.5 and 35 mg/kg) into male SD rats.
Figure 6 illustrates mean plasma concentrations of Anagrelide in a pharmacokinetic in vivo experiment for 28 days after subcutaneous injection of silica hydrogel composite comprising Anagrelide HCl. Mean (average for 5 animals) plasma concentration of Anagrelide HCl as a function of time for 28 d experiment after subcutaneous injections (SC) of silica hydrogel composite #29HG (depot) comprising Anagrelide HCl (35 mg/kg) into male SD rats.

### DETAILED DESCRIPTION OF THE INVENTION

The present invention relates to a hydrogel composite formulation comprising at least one active pharmaceutical ingredient (API) as solid particles. The main gist of present invention is that although said solid particles of API remain as solid particles as they become encapsulated in the loose hydrogel part of the hydrogel composite together with a large amount of non-active silica particles, both controlled and sustained release behavior, and thin-needle injection properties are achieved.

### Terms

*Gel* should be understood in the present context to be a homogeneous mixture of at least one solid phase and one liquid phase, i.e., a colloidal dispersion, where solid phase(s), e.g., silica as such and/or as partly or fully hydrolysed, is the continuous phase and the liquid(s), e.g. water, ethanol and residuals of silica precursors, is homogeneously dispersed in the structure. The gel is viscoelastic and the elastic properties dominate at rest, which is indicated by rheological measurements under small angle oscillatory shear. The elastic properties dominate, and the structure is non-flowing when the loss factor (or the loss tangent), tan δ = (G7G'), is less than 1. The combined effect of the elastic modulus G' and the viscous modulus G" can also be expressed in the form of complex modulus (or complex shear modulus), G*=G' + iG".

*Gel point* or *Gelation* shall be understood to mean the point when the sol that is flowing turns to a gel that is non-flowing and viscoelastic and the elastic properties dominate, which is indicated by rheological measurements under small angle oscillatory shear that the elastic modulus, G' is greater than the viscous modulus and the loss factor is less than 1. The viscoelastic properties are commonly measured with a rheometer (a measuring device for determination of the correlation between deformation, shear stress and time) by the oscillatory shear, where shear stresses are small (small angles of deformation). The measurements are conducted by ensuring an adequate signal for a specific measuring system, i.e., a strain sweep is commonly done at constant frequencies to find the proper signal and the linear viscoelastic region for the rheometer system and then the actual measurements are done at constant strain with varying frequency. The varying frequencies give varying elastic and viscous modulus and the measurement show whether the solid or liquid phase dominates. In the form of a sol, the liquid state dominates, but the system contains varying amounts of solid phase(s) and the system is still flowing. Before the gel point it is typical that a steep increase in dynamic viscosity and elastic modulus is observed, which continues to rise after the gel point as the structure is developing. In the context of the present invention gel point of the composite of the invention has been reached prior to obtaining the injectable gel of the invention.

*Non-active silica particles* refers to silica particles as such, i.e., they do not comprise any encapsulated or embedded agents (other than possible residuals originating from the synthesis of silica, such as water and ethanol), such as active pharmaceutical ingredients. Non-active silica particles are thus free of any active pharmaceutical ingredients. *Active silica particles* refers to silica particles comprising from 0.1 to 70 weight-%, preferably from 0.3 to 50 weight-%, and most preferably from 1 to 20 weight-% of an active pharmaceutical ingredient. All material properties defined for non-active silica particles, such as particle size and weight-% of the total solid content of the silica hydrogel composite, are valid also for active silica particles.

*Non-flowing and structurally stable when stored at rest* refers to the stable composite hydrogel structure which is comprised of non-active silica particles and API particles in the silica hydrogel. The stability is indicated by rheological measurements under small angle oscillatory shear by the elastic modulus, G' that is greater than the viscous modulus and the loss factor that is less than 1. When the elastic modulus is greater than the viscous modulus and the loss factor is less than 1, the structure is non-flowing. The non-flowing structure ensures the stability of the composite hydrogel structure by preventing the phase separation of the particles. In other words, the non-active silica particles and API particles are embedded in the silica hydrogel and they do not, e.g., precipitate or separate on the bottom of a vessel, e.g., a syringe, where the hydrogel composite is stored, typically at temperatures ≤ 25 °C. Although the composite hydrogel structure is non-flowing as stored at rest, e.g., in a prefilled, ready-to-use syringe, the structure is so loose that it is shear-thinning, and hence injectable through thin needles, as shear stress is applied on the hydrogel composite by injection.

The *hydrogel* should be understood to be a gel, where the liquid phase is water or where the liquid phase is water-based and contains more than 50 weight-% (wt-%) of water. Preferably the liquid phase of the hydrogel comprises > 80 wt-%, more preferably > 90 wt-% and even more preferably > 97 wt-% of water. The liquid phase can additionally comprise other liquids, typically organic solvents, e.g. ethanol. Typically, the concentration of such solvents, e.g. ethanol, is < 10 wt-%, more preferably < 3 wt-% and even more preferably < 1 wt-%. In the context of this invention the composite of the invention is considered a hydrogel since it fulfils the basic criteria of a hydrogel. Accordingly, when referring to *the hydrogel composite* of the invention this referral is equivalent to a referral to the composite of the invention. In the context of this invention the silica hydrogel composite of the invention preferably comprises 20 to 80 wt-%, more preferably 30 to 70 wt-%, and most preferably 40 to 60 wt-% of water.

The sol should be understood to be a homogeneous mixture of at least one liquid phase and one solid phase, i.e., a colloidal dispersion, where the liquid phase(s), e.g. water, ethanol and residuals of silica precursors, is the continuous phase and the solid phase(s), e.g. colloidal particles of silica and/or as partly or fully hydrolysed silica and/or aggregates of said particles are homogeneously dispersed in the said liquid phase characterized in that the sol has clear flow properties and the liquid phase is dominating.

*Injectable Gel or Hydrogel or Hydrogel Composite* in a context of this application is a rheological property of a composition. Before injection, e.g., as stored in a syringe and/or in an aluminium foil at temperatures < 37 °C, e.g., at room temperature (at 20-25 °C), or at refrigerator temperatures (at 2-8 °C) the composition is a gel, i.e., the elastic modulus (measured under small angle oscillatory shear) G' is greater than the viscous modulus G" and the loss factor, tan δ = (G"/G'), is less than 1. Although the hydrogel composite structure is a gel-like structure and the composite structure remains stable and non-flowing as stored at rest, the gel structure is so loose that it is shear-thinning when shear stress, e.g., in the form of injection through a needle from a syringe is applied, e.g., by using 18-25G needle (outer/inner diameter from 1.27/ 0.84 mm to 0.50/0.26 mm).

*Injectable* means, in the context of this invention, administrable via a surgical administration apparatus, e.g. a needle, a catheter or a combination of these.

*Shear-thinning* in the context of this application is a rheological property of a composition. Whenever the shear stress or shear rate of such a composition is altered, the composition will gradually move towards its new equilibrium state and at lower share rates the shear thinning composition is more viscous, and at higher shear rates it is less viscous. Thus shear-thinning refers to an effect where a fluid's viscosity, i.e. the measure of a fluid's resistance to flow, decreases with an increasing rate of shear stress.

*Matrix material* should be understood to be a material, such as silica microparticles or silica hydrogel composite, in which active pharmaceutical ingredient is incorporated, added, encapsulated or embedded, and matrix material due to its structure, such as pore structure and/or due to its chemical composition, such as dissolution in body fluids, controls the release rate of active pharmaceutical ingredient.

*Dosage form* should be understood to be injectable or implantable formulation comprising the matrix material, which is used in administration of active pharmaceutical ingredient.

*Depot formulation* referred to in the application is defined to be the administration of a sustained-action drug (active pharmaceutical ingredient) formulation that allows slow release and gradual absorption, so that the active agent can act and is released in the body for longer periods of times, i.e., from several days to several months. Depot formulations are administered parenterally, either by subcutaneous, intramuscular, peritoneal or ocular injection or implantation.

The term *silica* refers to amorphous SiO₂ that is preferably prepared by a sol-gel process. The sol-gel derived silica refers to silica prepared by the sol-gel process wherein the silica is prepared from liquid phase precursors, such as alkoxides, alkylalkoxides, aminoalkoxides or inorganic silicate solutions, which by hydrolysis and condensation reactions form a sol that turns to a gel or forms a stable sol. The sol-gel derived silica can also be prepared by processing to different morphologies by simultaneous gelling, aging, drying and form-giving, e.g. by spray-drying to microparticles.

The term *silica sol* refers to a suspension, i.e., mixture of a liquid (the continuous phase) and a solid phase (the dispersed phase), where the solid phase is comprised of silica particles and/or aggregated silica particles, where the particle size of the silica particles and/or aggregates is typically below 1 µm, preferably below 100 nm, i.e., the silica particles and/or particle aggregates are colloidal. A silica sol is commonly prepared from alkoxides or inorganic silicates that, via hydrolysis, form either partly hydrolysed silica species or fully hydrolysed silicic acid. The liquid phase is typically comprised of water and hydrolysis and condensation products, such as ethanol. Subsequent condensation reactions of SiOH-containing species lead to formation of larger silica species having an increasing amount of siloxane bonds. These species form nanosized, colloidal particles and/or particle aggregates. Depending on the conditions the silica sol remains as a stable colloidal suspension or it turns into a gel.

*Active pharmaceutical ingredient (API)* should be understood to be a drug or other therapeutic and/or biologically active agent, which is preferably poorly water soluble or completely water insoluble. Solid particles of API comprise essentially API, and they are free of silica. Preferably a solid particle of API comprises at least 80 weight-%, preferably 90 weight-%, more preferably 95 weight-% or 99 weight-%, sometimes even 99.5 weight-% or more of API.

*Particle size* or *particle diameter* refers to the largest diameter of a particle when particle is of arbitrary form.

*R value* refers to molar ratio water-to-tetraethyl orthosilicate (TEOS), e.g., R300 corresponds to molar ratio water-to-tetraethyl orthosilicate = 300. TEOS is a common precursor for sol-gel derived silica and it is also used in the present invention. R value can also be used to calculate the solid (silica) content for a silica sol. For example, molar ratio water-to-TEOS of 200 (R200) results in ca. 1.60 weight-% of solid silica in a silica sol, R300 correspondingly in ca. 1.08 weight-%, and R400 correspondingly in ca. 0.82 weight-%.

*Micronization* should be understood in the context of the present invention to be any method used to produce small solid particles, e.g., solid particles of active pharmaceutical ingredients, as long as the resulting particle size of solid matter is 300 micrometers or less. For example, solid particles of API can be produced by 1) direct synthesis and/or precipitation, or 2) by any crystallization method or supercritical fluid technology for small particles, such as controlled expansion of supercritical solution, or 3) by any dissolution-precipitation/crystallization cycle, or 4) by spray- or freeze-drying, or 5) by any mechanical method, such as grinding, bashing, and milling, used to decrease size of solid matter, such as grinding by mortar and pestle, wet milling, pneumatic milling, or 6) by any granulation method starting from submicron particles.

### Features of the invention

The present inventors have found that a silica hydrogel composite comprising a hydrogel part, non-active silica microparticles and API as solid particles is able to achieve both desirable controlled-release properties and thin-needle injection from prefilled syringes. The API can be added into silica hydrogel part as solid particles, and due to their very low solubility in water, the API particles also remain as solid particles when present in the hydrogel part of the composite. The hydrogel composite comprises also non-active silica microparticles, i.e. silica microparticles without encapsulated or embedded API. The non-active silica microparticles adjust the rheological properties of the hydrogel composite, but they also contribute to the overall controlled release of API by preventing the release of solid particles of API that are embedded in the hydrogel part of the hydrogel composite. The hydrogel part and the non-active silica microparticles thus form together a non-flowing, but injectable silica hydrogel composite, where the solid particles of API are incorporated and/or embedded. In this manner the API is not released from the hydrogel composite without dissolution of the solid phase of the hydrogel composite.

In the present context the term "hydrogel part" refers to the part of the hydrogel composite that originates from the silica sol.

The present invention provides a silica hydrogel composite comprising at least one active pharmaceutical ingredient (API) as solid particles. In the manufacture of silica hydrogel composite, the API is in form of a dry powder or as a suspension of solid particles prior to its encapsulation or incorporation into the silica hydrogel. As noted above, one of the surprising findings of present invention is that although said solid particles of API remain as solid particles, preferably having a diameter ≤ 300 µm, after their encapsulation/incorporation in the loose hydrogel part of the silica hydrogel composite, both controlled and sustained release behavior in vitro and in vivo, and injection from syringes with thin needles, such as 18G-25G needles, are still achieved for the silica hydrogel composite.

The silica hydrogel composite comprises non-active silica particles and solid particles of at least one API in the hydrogel part of the composite, and they both affect the controlled release properties. The controlled release properties are thus not only influenced by the hydrogel part of the composite and the non-active silica particles, but also the particle size of the solid particles of active pharmaceutical ingredient. It is surprisingly found that practically water insoluble or poorly water soluble active pharmaceutical ingredient can be used as solid particles to prepare a homogeneous silica hydrogel composite for controlled release with thin needle injectability. The particle size of the solid particles of API may be ≤ 300 µm, preferably from 1 µm to 300 µm, more preferably from 1 µm to 200 µm.

Different gels, such as organogels and hydrogels have conventionally been used in controlled drug delivery, but there are many challenges with respect to different types of active pharmaceutical ingredients. Hydrogels, as gels in general, have typically a low solid content and a major part of the gel structure is in liquid form. The solid part or solid phase of a hydrogel comprises polymeric molecules or colloidal species, e.g., nanoparticles in a sol, that crosslink, agglomerate or aggregate to form three-dimensional networks. Water-based liquid is homogeneously distributed in the formed three-dimensional network. In practice, a hydrogel is a porous structure, where the pores are filled with a water-based liquid. The solid content of a hydrogel is typically low, such as ≤ 3 weight-%, and in many cases lower than ≤ 1 weight-%. At low solid content, the hydrogel turns easily into flowing and shear-thinning material, which is beneficial when developing injectable dosage forms for controlled drug delivery. When the solid content of a hydrogel increases, the shear-thinning property may be lost. Because a hydrogel structure is typically a quite loose and open structure, incorporated, encapsulated or embedded active pharmaceutical ingredients of different size, such as small-molecule drug molecules, proteins, peptides, and RNA, have a possibility to diffuse out relatively fast. If the release of an encapsulated API from a hydrogel is dependent on the dissolution rate of the solid phase, the size of API must be larger than the pores in the hydrogel network. However, the release rate is also dependent on the total solid content of a hydrogel that is typically low, especially for typical dosage forms for a thin-needle injection. Low solid content of a hydrogel, such as less than 3 weight-%, which is also feasible also for thin-needle injections, may still be too loose even for larger active pharmaceutical ingredients or therapeutic agents, such as solid API particles, fusion proteins, viral vectors and vaccine antigens to reach sustained delivery. In the present invention the non-active silica particles are combined with a silica hydrogel and solid API particles, which results in the silica hydrogel composite, where both good injectability with thin needles and sustained delivery is achieved.

In the present invention the silica hydrogel composite comprises non-active silica particles and solid particles of at least one API embedded in a silica hydrogel. The silica hydrogel composite is especially suitable for controlled release of APIs which have poor solubility in water or which are completely insoluble in water because the main component of the liquid phase in the silica hydrogel is water. The silica hydrogel part of the silica hydrogel composite is a loose hydrogel with the solid content preferably ≤ 3 weight-%, preferably ≤ 2 weight-%, most preferably 0.5 - 2 weight-%.

The solid content in the silica hydrogel part is typically less than 1.5 weight-% of the total silica hydrogel composite.

In the present invention the silica hydrogel composite comprises non-active silica particles having a diameter of ≤ 100 µm, or in a range from 1 µm to 100 µm, preferably from 1 µm to 30 µm, more preferably from 1 µm to 20 µm. The use of non-active silica particles as a part of the silica hydrogel composite increases the solid content of the hydrogel composite without any loss of the thin needle injectability, and the non-active silica particles also contribute to the sustained release rate of the solid particles of the API. The non-active silica particles may be spray dried silica particles or silica fiber fragments. Alternatively, the non-active silica particles may be moulded or casted silica monoliths, as such or as crushed.

The silica hydrogel composite comprises up to 75 weight-% of said non-active silica particles, and according to one embodiment of the invention 20-75 weight-% of the total silica hydrogel composite is non-active silica microparticles. According to one embodiment the silica hydrogel composite may also comprise active silica particles, and in that case the total portion of said non-active and active particles is also 75 weight-%. The general role of the non-active silica particles is to achieve good rheological properties for the dosage form, i.e., stable hydrogel composite structure at rest (e.g., in a syringe) and shear-thinning properties under shear (e.g., when injecting the hydrogel composite from a syringe through a needle, such as 18-25G).

The silica hydrogel composite may have a total solid content from 20 weight-% to 80 weight-%, preferably from 30 weight-% to 60 weight-%, even more preferably from 35 weight-% to 55 weight-%.

The silica hydrogel composite comprises at least one active pharmaceutical ingredient (API) as solid particles. It is also possible that the silica hydrogel composite comprises two or several different active pharmaceutical ingredients as solid particles. Preferably the diameter of the solid particles of API is ≤ 300 µm, more preferably in the size range of 1-200 µm, The solid particles of at least one API encapsulated in the hydrogel part of the silica hydrogel composite results in an injectable dosage form, where a sustained release is achieved despite of the encapsulation of the solid particles in the loose hydrogel part of the hydrogel composite. The water solubility of active pharmaceutical ingredient is preferably low or non-existent, and the both low solubility and the size of the solid particles of API contribute to the sustained and controlled release achieved with the silica hydrogel composite of the present invention. The larger the size of the solid particles of API, the more the particle size affects the release rate of the API. However, the solid particles of active pharmaceutical ingredients may also affect the final gelation of the silica hydrogel composite as well as its rheological properties, and thus, the optimal amount and particle size of the solid particles of API, is preferably to be decided case by case, depending on the API in question. According to one embodiment 0.1-20 weight-%, preferably 0.1-15 weight-%, of the total silica hydrogel composite may be solid particles of API. For example, if the API is Anagrelide HCl, the particle size of the solid particles of API is 1-300 µm, preferably 1-200 µm, and/or the amount of the solid particles of API is 0.1-20 weight-%, preferably 0.1-15 weight-%, calculated from the total silica hydrogel composite.

Solid particles of at least one API, having preferably a diameter of ≤ 300 µm, more preferably 1-200 µm, can be easily added to liquid systems that form a hydrogel, e.g., into a silica sol that is used to form a silica hydrogel, or into a silica sol that will form the hydrogel part in a silica hydrogel composite comprising also non-active silica particles. According to one embodiment of the invention the particle size of the solid particles of API may be 100-1000 nanometers. The solid particles of API are embedded in the silica hydrogel part, without losing good injectability of the formed hydrogel composite, which is an important property for minimally invasive dosage forms in controlled drug delivery. When solid particles of the API are clearly larger than 1 micrometer, the homogeneous distribution of the particles must be ensured by mixing until the system has turned from a flowing form, e.g., from a silica sol, to a non-flowing form, e.g., into silica hydrogel, or into silica hydrogel composite. API particles of the size from 1 to 300 µm can be prepared by any method of micronization, such as direct synthesis and/or precipitation.

Both good injectability, for example with 18-25G needles, of the silica hydrogel composite, and controlled release in vitro and in vivo are achieved with the silica hydrogel composite comprising solid particles of API with the size of 300 µm or less or with 1-200 µm. The controlled and sustained release property with the solid particles of API particles, embedded in the hydrogel part of the silica hydrogel composite is achieved although the silica hydrogel part has a loose structure with low solid content. This is due to combined effect of the hydrogel part, large size of the API particles and non-active silica particles. The silica hydrogel part and non-active silica particles together form a structure, where the solid particles of API are entrapped so that they are not released fast as particles, but slowly when the major solid component of the silica hydrogel composite, i.e. non-active silica particles dissolve. Silica itself does not dissolve inside the silica hydrogel composite, because it has a very low water solubility (e.g., 120-150 ppm (micrograms/ml) at 37 °C and pH7.4, and even lower at room temperature or at 2-8 °C), i.e. the silica dissolves mostly from the surface of the hydrogel composite when in vitro dissolution medium is refreshed to keep it in sink conditions (conditions ensuring free dissolution of silica) or when the body fluids flow in vivo. The portion of the non-active silica microparticles of the silica hydrogel composite is high, preferably up to 75 weight-%, or 20-60 weight-%, and the corresponding portion for aggregated silica sol nanoparticles, which form the solid phase in the hydrogel part of the hydrogel composite, is typically less than 3 weight-%. The rest is aqueous solution and encapsulated solid particles of active pharmaceutical ingredient (e.g. 0.1-20 weight-%). Thus, the non-active silica particles form the major part of the solid phase in the final hydrogel composite. As aggregated silica sol nanoparticles in silica sol (which also comprises the aqueous solution), non-active silica particles, and solid particles of active pharmaceutical ingredients are combined, they form together a silica hydrogel composite, where the solid particles of active pharmaceutical ingredient become encapsulated between the silica particles (non-active silica particles and aggregated silica nanoparticles) and the aqueous solution is homogeneously distributed throughout the whole mass of the hydrogel composite.

The slow release is supported by the poor or no solubility of the API particles, i.e., their size does not decrease, and they stay entrapped in the hydrogel composite structure. According to one preferable embodiment the effect of the large size of the solid particles of API on good controlled release properties was observed for solid particles of Anagrelide HCl with solubility of 0.019 mg/ml in water corresponding to 0.0019 weight-%, which means that solid particles of API remained practically intact in the hydrogel part of the composite in a typical syringe dose of 0.05-1 ml in parenteral administration. It can be assumed that any solid particle of API with size less than 300 micrometers dissolving less than 10 weight-% in the aqueous phase of one dose of the silica hydrogel composite retain their positive effect both on thin-needle injection and on the controlled release properties. The good sustained and controlled release behavior in the present invention was observed for API particles having a relatively large particle size distribution between 1 and 200 micrometers, i.e., the average size difference for the smaller half of the particles compared with the larger half of the particles was more than 10%. If 10 weight-% is dissolved in the aqueous phase of the hydrogel phase, it means in practice that 90 weight-% of the API particles are still intact, and possible faster initial release of API (a burst) of 10 weight-% due to dissolved form API is generally within limits of acceptance in controlled drug delivery.

According to one embodiment of the invention the composition may further comprise active silica particles comprising from 0.1 to 70 weight-%, preferably from 0.3 to 50 weight-%, and most preferably from 1 to 20 weight-% of an active pharmaceutical ingredient. It is possible to encapsulate API as nanoparticles of particle size 10-100 nm into active silica particles prepared, e.g., by spray-drying, and use them in the silica hydrogel composite, as such instead of non-active silica particles, or preferably in addition to non-active silica particles. In case the silica hydrogel composite comprises both non-active and active silica particles, the total amount of silica particles, including both active and non-active, does not exceed the maximum weight-% of silica particles of 75 weight-% of the total mass of the silica hydrogel composite, as defined elsewhere in this application. If the API particles are small enough, e.g. in the range of 10-100 nm compared with the matrix material size in the final dosage form, e.g., compared with spray-dried silica particles that are typically of the size 1-30 micrometers, it is possible that they are homogeneously encapsulated into silica particles and form active silica particles. The properties of the active silica particle properties (adjustable dissolution rate of silica) control the release properties of API from the active silica microparticles. The API encapsulated into active silica microparticle may be same or different than the API present as solid particles.

The size of API containing particles is one of the major parameters. Matrix materials used in drug delivery systems are many, and they are in many different forms, size, and shapes, such as monolithic implants, fibers, particles in suspensions, hydrogels etc. Microparticles are quite common, and they can be used as active silica particles. In that case encapsulated or embedded API particles as such should be small enough compared with the microparticles (typical size range of ca. 1-30 micrometers), i.e., API particles should be nanoparticles with a diameter below 100 nm. Too large API particles cannot be properly encapsulated in active silica particles, because it results heterogenous structure and they lose their controlled delivery properties. In addition, the loading-% of API in a matrix material may remain low if API particles are too large compared with the matrix particles. The same applies also to different kind of solid implant structures and fibrous materials, where API as large particles may cause heterogeneity and loss in controlled release properties, and in mechanical properties.

The silica hydrogel composite of the present invention comprising at least one active pharmaceutical ingredient as solid particles has advantages when developing new matrix materials for controlled and sustained delivery. In addition to the already presented option for use of relatively large solid particles of active pharmaceutical ingredient in the silica hydrogel composite for controlled delivery, there are also other options, which have been contemplated by the present inventors. For example, active pharmaceutical ingredient could be pre-encapsulated in some other material (in the form solid particles), which is not suitable for sustained release of the encapsulated agent, but it makes the encapsulation or embedment of active pharmaceutical ingredients with low water solubility easier into another material, e.g., into a matrix material, which controls the release of active pharmaceutical ingredients. Alternatively, or in addition, the pre-encapsulation may also be related to the protection of active pharmaceutical ingredients, such as small-molecule drugs or biological drugs (e.g., protein-based drugs, fusion proteins, peptides, RNA-based drugs, viral vectors, and vaccine antigens). Yet another possibility is to achieve more options for controlled and sustained delivery of active pharmaceutical ingredients (independently whether they are well soluble in water or other processing liquids for matrix materials or not), because release rate profiles from any matrix material are different if active pharmaceutical ingredients are present as large solid particles, small particles, or dissolved in the molecular form, or in different combinations of particles of different size and dissolved form.

### Preferred embodiments

In preferred depot formulations of the present invention the silica hydrogel composite comprises at least one active pharmaceutical ingredient as solid particles having preferably a diameter of ≤ 300 µm, and the silica hydrogel composite is non-flowing and structurally stable when stored at rest and shear-thinning when shear stress is applied by injection.

In depot formulations of the present invention the silica hydrogel composite comprises up to 75 weight-% of non-active silica particles.

In preferred depot formulations of the present invention the silica sol has a solid content of 0.5 - 3 weight-%, preferably 0.5 - 2 weight-%.

In preferred depot formulations of the present invention the non-active silica particles have a diameter in a range from 1 µm to 100 µm, preferably from 1 µm to 30 µm, more preferably from 1 µm to 20 µm.

In preferred depot formulations of the present invention the silica sol comprises silica sol particles having a diameter ≤ 100 nm, more preferably from 5 to 100 nm.

In preferred depot formulations of the present invention the silica hydrogel composite has a solid content from 20 weight-% to 80 weight-%, preferably from 30 weight-% to 60 weight-%, even more preferably from 35 weight-% to 55 weight-%.

In preferred depot formulations of the present invention the silica hydrogel composite further comprises active silica particles comprising from 0.1 to 70 weight-%, preferably from 0.3 to 50 weight-%, and most preferably from 1 to 20 weight-% of an active pharmaceutical agent.

In preferred depot formulations of the present invention silica is an alkoxysilane-derived silica, preferably tetraethoxysilane-derived silica.

In preferred depot formulations of the present invention the non-active silica particles are selected from the group consisting of spray dried silica particles; silica fiber fragments; and moulded or casted silica monoliths, as such or as crushed.

In preferred depot formulations of the present invention the solid particles of active pharmaceutical ingredients are selected from a group consisting of particles prepared by direct synthesis and/or precipitation; crystallization methods or supercritical fluid technology for small particles, such as controlled expansion of supercritical solution; dissolution-precipitation/crystallization cycle; spray- or freeze-drying, mechanical methods, such as grinding, bashing, and milling, used to decrease size of solid matter, such as grinding by mortar and pestle, wet milling, pneumatic milling; granulation methods starting from submicron particles.

In preferred depot formulations of the present invention, the silica hydrogel composite is formed at least by i) aggregated silica sol nanoparticles in silica sol (which also comprises the aqueous solution), ii) non-active silica particles, and iii) solid particles of active pharmaceutical ingredients, wherein the solid particles of active pharmaceutical ingredient become encapsulated between the silica particles (non-active silica particles and aggregated silica nanoparticles) and the aqueous solution is homogeneously distributed throughout the whole mass of the hydrogel composite.

The depot formulation of the present invention is typically used for administering an active pharmaceutical ingredient.

The depot formulation of the present invention is used for administration of Anagrelide or any of its pharmaceutically accepted salts, including the hydrochloride salt.

The depot formulation of the present invention is typically used for parenteral administration.

The depot formulation of the present invention is typically used for parenteral administration by injection.

The depot formulation of the present invention is typically used for controlled delivery of an active pharmaceutical ingredient.

### EXAMPLES

Some embodiments of the present invention are described in the following nonlimiting examples.

### Example 1

### Preparation of Silica hydrogel composites comprising Anagrelide HCl (API)

A silica sol for the preparation of non-active silica particles (plain silica microparticles) was started by hydrolysis of TEOS in water using 0.1 M HCl as a catalyst at pH 2. The molar water-to-TEOS ratio =R value) of the silica sol was 2.5 (the first R value). After the hydrolysis, the R2.5 sol was cooled down to 0-5 °C. Then the R2.5 sol was diluted with ethanol to decrease the solid (silica) content in the sol ending up with the second R value of 50 (added ethanol volume corresponds to volume of water that is needed to obtain molar water-to-TEOS ratio of 50). Lastly, the pH of the diluted silica sol (R2.5-50) was adjusted with a 0.1 M NaOH solution to pH4.9 prior to spray-drying. Non-active silica particles were then prepared by spray-drying (using Büchi B-290 spray-dryer) the R2.5-50 silica sol. The spray-drying parameters are listed in Table 1.

**Table 1. Spray-drying parameters**

| **Inlet temperature** | **Outlet temperature** | **Aspirator** | **Pump** | **Atomization air flow** |
|---|---|---|---|---|
| 120 °C | 83-85 °C | 35 m³/h | 5.6 ml/min | 670 l/h |

Because Anagrelide HCl is practically insoluble in water (0.019 mg/ml), and the base form of Anagrelide has even lower solubility than Anagrelide HCl, the solid Anagrelide HCl powder was micronized (to particle size distribution of 1-200 micrometers) manually in a mortar for ca. 10 minutes.

Next step was the preparation of the hydrogel part of the silica hydrogel composite out of a R200 or a R300 silica sol. Both R200-based and R300-based sols (molar ratio water-to-TEOS of 200 (R200) results in ca. 1.60 weight-% of solid silica in a silica sol, and R300 correspondingly in ca. 1.08 weight-%) were successfully used to prepare an injectable silica hydrogel composite. R300 silica sol was prepared by hydrolysis of TEOS in deionized water at pH2 using 0.1 M HCl as a catalyst. Non-active silica particles spray-dried from R2.5-50 sol, micronized API powder (Anagrelide HCl ) and R300 silica sol were combined by suspending 920 mg of micronized API (Anagrelide HCl ) and 6780 mg of non-active silica particles (R2.5-50) in 12.6 ml of R300 silica sol followed by addition of 0.5M NaOH (6.1 ml) to adjust the pH of the mixture to pH5.8. The mixture was transferred into plastic syringes (Becton Dickinson luer-lock, 1 ml syringes without needle), the mixture was kept stable (sedimentation of non-active silica particles and solid Anagrelide HCl particles was prevented) in syringes by gentle mixing in a vertical rotating mixer and the mixture was allowed to gel at ambient temperature (ca. 25 °C) for 2-3 days until gelation occurred, i.t., the mixture turned into a non-flowing silica hydrogel composite #29HG.

### Example 2

*In vitro dissolution and release rate measurements for non-active silica microparticles and for silica hydrogel composites comprising Anagrelide HCl (API)* The in vitro degradation of silica and the release of the API (Anagrelide HCl) was measured in 50 mM Tris buffer, pH 7.4 at 37 °C supplemented with SDS (0.5 %, w/v). The sample size analyzed was ca.10-15 mg for non-active silica particles and ca. 20-30 mg for silica hydrogel composite (depot) formulation. The dissolution tests were conducted up to 72 hours in a shaking water bath (60 strokes/min) at 37 °C. Silica and the API (Anagrelide HCl) concentrations were kept at in sink condition (free dissolution of the silica matrix, i.e., silica concentration is kept below 20 % of the saturation concentration) in the dissolution medium. The dissolution medium was changed to fresh medium at every sampling time point in order to keep the silica concentrations below 30 ppm (in sink condition). A quantitative analysis was used to measure the cumulative API release and silica dissolution. Three replicate samples were collected at each time point and mean values are shown in the results. Silica concentrations were measured with UV/VIS-spectrophotometer analyzing the molybdenum blue complex absorbance at λ = 820 nm. Anagrelide was analyzed with a high-performance liquid chromatograph (HPLC) 1100 HPLC Agilent Technologies connected to variable wavelength detector (at λ = 250 nm). The chromatographic separation was obtained on a Waters XSelect HSS C18, 3.5 µm, 3.0x20 mm HPLC column, with a Model G1314A Variable wavelength detector at 1100 HPLC Agilent Technologies, at column temperature of 30 °C and with water/Formic acid 1000/1 (v/v) as mobile phase A and Acetonitrile/Formic acid 1000/1 (v/v) as mobile phase B.

The total silica and Anagrelide HCl contents in the hydrogel composites were measured in order to determine the dissolution and release rates accurately. The total silica content of the sample materials was measured by dissolving samples for 3 days in 0.5 M NaOH solution at 37 °C. Correspondingly, total API (Anagrelide HCl) content was measured by dissolving samples in 50 mM glycine buffer pH 9.6 at 37 °C supplemented with SDS (1.5% w/v).

The in vitro dissolution study for the non-active silica particles (R2.5-50, pH 4.9) was run up to 48 hours in sink conditions. In vitro cumulative silica degradation (i.e., dissolution of the silica matrix) from the non-active silica particle formulation reached 100 % (w/w) in ca. 24 hours, before that ca. 7 % at 1 h, ca. 32 % at 2 h, ca. 59% at 3 h, ca. 68 % at 4 h, ca. 77% at 5 h, ca. 83 at 6 h, ca. 95 % at 9 h.

Cumulative degradation (dissolution rate) of silica and the cumulative release of Anagrelide from silica hydrogel composite depot formulation #29HG is shown in Figure 1. The dissolution study was run up to three days in sink conditions. The silica dissolution rate from the silica hydrogel composite #29HG (comprising non-active silica particles R2.5-50, pH 4.9) was a slower than that for non-active silica microparticles (R2.5-50, pH 4.9), as such. The same dissolution experiment for silica hydrogel composite depot formulation #29HG, but now calculated to correspond to mg/hour release for dose of 20 mg of Anagrelide HCl in the silica hydrogel composite, is shown in Figure 2.

### Example 3

*Rheological measurement and injectability for silica hydrogel composites and particle size analysis for non-active silica particles and Anagrelide HCl particles* Rheological measurements were conducted with a rotational rheometer (Haake RheoStress 300, Germany), equipped with a parallel plate-plate measuring geometry (D = 20 mm). Two different rheological characteristics were studied: dynamic viscosity as a function of shear rate and viscoelasticity. The dynamic viscosity of the silica hydrogel composites was measured under a controlled shear rate (CR) rotatory ramp -program with a gap of 0.2 mm and shear rates ranging from 100 1 /s to 6000 1/s at 25 °C. The viscoelastic properties of the samples were studied via oscillatory measurements within the linear viscoelastic region (determined with an amplitude sweep measurement) of the samples with a measuring gap of 0.4 mm. The samples were studied under controlled deformation (γ < 0.002) within a frequency range of 0.01-10 Hz.

Injectability was tested by injecting depot using a plastic 1 ml luer-lock syringe (Becton Dickinson, 1 ml syringe without a needle) connected to different sized needles. Injection volume was 200-300 µl.

The oscillatory measurements (Figure 3) showed that depot formulation #29HG had a non-flowing gel-like structure at low frequencies, suggesting that the non-active silica particles and API particles do not sediment within silica hydrogel composite (the depot) at rest (e.g., as stored in a syringe). Loss factor (tan δ = G"/G"), which is the relation between viscous (loss) modulus (G") indicating the liquid-like properties of a viscoelastic material and elastic (storage) modulus (G') indicating solid-like properties of a viscoelastic properties, show clearly that the solid-like properties dominate at rest, i.e., it is a non-flowing material. G' is ca. 10-20 larger than G", and G' is ca. 415-440 kPa at the studied frequencies indicating a relatively rigid silica hydrogel composite structure. However, the dynamic viscosity measurement indicated that the depot formulation had clear shear-thinning properties, predicting injectability through thin needles (20G). Dynamic viscosity for the depot formulation #29HG decreased clearly from ca. 20 Pas at shear rate of ca. 1 1/s to ca. 48 mPas at shear rate of ca. 1500 1/s. The injectability of the depot formulation was further assessed by conducting a manual injectability test which showed that the depot formulations could be injected through a thin needles (20G).

For particle size distribution analysis, the non-active silica particles (which were used in silica hydrogel composite #29HG) were dispersed in ethanol, and the analysis was conducted using a Sympatec HELOS H3973 laser diffraction instrument. The particle size distribution for non-active silica particles is shown in Table 2 (D10, D50 and D90 means that 10 %, 50 %, and 90 % of the particles are equal or smaller than the indicated size)

**Table 2. Particle Size Distribution of non-active silica particles**

| **Formulation** | **D10 (µm)** | **D50 (µm)** | **D90 (µm)** |
|---|---|---|---|
| non-active silica | 1.33 ± 0.02 µm | 3.22 ± 0.11 µm | 6.89 ± 0.28 µm |

The particles size distribution analysis for micronized (described in Example 1) Anagrelide HCl particles was also conducted using a Sympatec HELOS H3973 laser diffraction instrument. The particle size was mainly between 1 and 200 micrometers, where D10 was 16.6 micrometers, D50 80.4 micrometers, and D90 155 micrometers.

### Example 4

### In vivo pharmacokinetic experiments for silica hydrogel composites comprising solid particles of Anagrelide hydrochloride

Silica hydrogel composite #29HG was used as a depot for male SD (Sprague Dawley) rats (5 animals/dosing group) in subcutaneous (SC + a letter A-E referring to different groups) administration of Anagrelide, and for oral administration (PO). The oral dose was prepared by adding 18.00 mg of Anagrelide hydrochloride into 31.520 ml of 10 w-% ethanol with vortexing and sonication to obtain a suspension with concentration of 0.5 mg/ml of Anagrelide hydrochloride. The oral dose (5 mg/kg) was provided only in 12 h study, and it was freshly made just prior to dosing. Both 12 hour and 10 d pharmacokinetic experiments were conducted for the silica hydrogel composite for 2 different anagrelide hydrochloride doses (17.5 mg/kg and 35 mg/kg), and 28 d experiment only for the dose of 35 mg/kg. The doses, administration routes and blood sampling times are shown in Table 3.

**Table 3. Parameters of the in vivo pharmacokinetic experiment for Anagrelide HCl treatment**

| **Group** | **Dose Level (mg/kg)** | **Dose Volume (ml/kg)** | **Volume (mg/ml)** | **Route** | **No. of Animals** | **Blood sampling time points** |
|---|---|---|---|---|---|---|
| 1 | 5.0 | - | - | PO | 5 | 0, 0.5, 1, 2, 4, 6, 12 h |
| 2 | 17.5 | 0.296 | 59.1 | SCA | 5 | 0, 0.5, 1, 2, 4, 6, 12 h |
| 3 | 17.5 | 0.296 | 59.1 | SCB | 5 | 0, 1 d, 3d, 7 d, 10 d |
| 4 | 35.0 | 0.592 | 59.1 | SCC | 5 | 0, 0.5, 1, 2, 4, 6, 12 h |
| 5 | 35.0 | 0.592 | 59.1 | SCD | 5 | 0, 1 d, 3 d, 7 d, 10 d |
| 6 | 35.0 | 0.592 | 59.1 | SCE | 5 | 0, 1h, 3d, 6d, 9d, 12d, 15d, 18d, 21d, 24d, 28d |

No abnormal clinical symptoms were observed in SD rats during the entire experiment. The bioanalysis was conducted by using LC-MS/MS. The desired serial concentrations of working solutions were achieved by diluting stock solution of analyte with 50% acetonitrile in water solution. 5 µL of working solutions (2, 5, 10, 20, 50, 100, 500,1000, 5000, 10000 ng/mL) were added to 50 µL of the male blank SD Rat plasma to achieve calibration standards of 0.2-1000 ng/mL (0.2, 0.5, 1, 2, 5, 10, 50, 100, 500, 1000 ng/mL) in a total volume of 55 µL. Four quality control samples at 0.5 ng/mL, 1 ng/mL, 50 ng/mL and 800 ng/mL for plasma were prepared independently of those used for the calibration curves. These QC samples were prepared on the day of analysis in the same way as calibration standards. 55 µL of standards, 55 µL of QC samples and 55 µL of unknown samples (50 µL of plasma with 5 µL of blank solution) were added to 200 µL of acetonitrile containing IS mixture for precipitating protein respectively. Then the samples were vortexed for 30 s. After centrifugation at 4 degree Celsius, 4700 rpm for 15 min, the supernatant was diluted 3 times with water, and 10 µL of diluted supernatant was injected into the LC-MS/MS system for quantitative analysis. The mean plasma concentrations of Anagrelide Hydrochloride for 12 h experiment are shown in Figure 4, and corresponding results for 10 d experiment in Figure 5, and for 28 d experiment in Figure 6.

The plasma concentrations results show that sustained and controlled release is achieved when delivering Anagrelide HC, which was used as solid particles in silica hydrogel composite.

**Table 4. Summary of Anagrelide HCl pharmacokinetic parameters for different doses**

| **Dose** | **T_{1/2}** h | **Tₘₐₓ** h | **Cₘₐₓ** ng/ml | **AUCₗₐₛₜ** h*ng/ml | **AUC_{last/D}** h*mg/ml |
|---|---|---|---|---|---|
| PO | 1.30 ± 0,27 | 3.00 ± 2.00 | 62 ± 47 | 320 ± 235 | 64 ± 47 |
| SCA | 4.92 ± 0.85 | 1.10 ± 0.55 | 4.55 ± 1.41 | 29.2 ± 7.1 | 1.67 ± 0.40 |
| SCB | - | 96 ± 102 | 3.58 ± 1.51 | 521 ± 218 | 29.8 ± 12.5 |
| SCC | 7.03 ± 1.04 | 0.80 ± 0.27 | 6.3 ± 4.2 | 34.5 ± 10.6 | 0.99 ± 0.30 |
| SCD | - | 154 ± 79 | 5.29 ± 2.28 | 749 ± 320 | 21.4 ± 9.1 |
| SCE | 167 | 188 | 4,81 | 1599 | 45,7 |

## Claims

1. A silica hydrogel composite comprising at least one active pharmaceutical ingredient, wherein the silica hydrogel composite is obtainable by mixing
a. non-active silica particles having a diameter of ≤ 100 µm measured by laser diffraction,
b. a silica sol, having a solid content in the silica sol less than 3 weight-%, and
c. solid particles of at least one active pharmaceutical ingredient (API), the particles having preferably a diameter of ≤ 300 µm,
wherein the silica hydrogel composite comprises up to 75 weight-% of said non-active silica particles and wherein the silica hydrogel composite is non-flowing and structurally stable when stored at rest and shear-thinning when shear stress is applied by injection.

2. The silica hydrogel composite according to claim 1, **characterized in that** the silica sol has a solid content of 0.5 - 3 weight-%, preferably 0.5 - 2 weight-%.

3. The silica hydrogel composite according to claim 1 or 2, **characterized in that** the non-active silica particles have a diameter in a range from 1 µm to 100 µm, preferably from 1 µm to 30 µm, more preferably from 1 µm to 20 µm.

4. The silica hydrogel composite according to claims 1, 2 or 3, **characterized in that** the silica sol comprises silica sol particles having a diameter ≤ 100 nm.

5. The silica hydrogel composite according to any of preceding claims 1-4, **characterized in that** the silica hydrogel composite has a solid content from 20 weight-% to 80 weight-%, preferably from 30 weight-% to 60 weight-%, even more preferably from 35 weight-% to 55 weight-%.

6. The silica hydrogel composite according to any of preceding claims 1-5, **characterized in that** the silica hydrogel composite further comprises active silica particles comprising from 0.1 to 70 weight-%, preferably from 0.3 to 50 weight-%, and most preferably from 1 to 20 weight-% of an active pharmaceutical agent.

7. The silica hydrogel composite according to any of the preceding claims 1-6, **characterized in that** silica is an alkoxysilane-derived silica, preferably tetraethoxysilane-derived silica.

8. The silica hydrogel composite according to any of preceding claims 1-7, **characterized in that** the non-active silica particles are selected from the group consisting of spray dried silica particles; silica fiber fragments; and moulded or casted silica monoliths, as such or as crushed.

9. The silica hydrogel composite according to any of preceding claims 1-8, **characterized in that** the solid particles of active pharmaceutical ingredients are selected from a group consisting of particles prepared by direct synthesis and/or precipitation; crystallization methods or supercritical fluid technology for small particles, such as controlled expansion of supercritical solution; dissolution-precipitation/crystallization cycle; spray- or freeze-drying, mechanical methods, such as grinding, bashing, and milling, used to decrease size of solid matter, such as grinding by mortar and pestle, wet milling, pneumatic milling; granulation methods starting from submicron particles.

10. The silica hydrogel composite according to any of claims 1-9 for administering an active pharmaceutical ingredient.

11. The silica hydrogel composite according to claim 10, **characterized in that** the active pharmaceutical ingredient is Anagrelide or any of its pharmaceutically accepted salts, including the hydrochloride salt.

12. The silica hydrogel composite according to claim 10 or 11, **characterized in that** administration is parenteral.

13. The silica hydrogel composite according to claim 12, **characterized in that** administration is by injection.

14. A silica hydrogel composite according to any of the claims 1-9 for use in controlled delivery of an active pharmaceutical ingredient.

## Patentansprüche

1. Silicahydrogel-Verbundstoff, der mindestens einen pharmazeutischen Wirkstoff umfasst, wobei der Silicahydrogel-Verbundstoff durch Mischen erhältlich ist von
a. nicht aktiven Silica-Partikeln, die einen durch Laserbeugung gemessenen Durchmesser von ≤ 100 µm aufweisen,
b. einem Silicasol, das einen Feststoffgehalt im Silicasol von weniger als 3 Gewichts-% aufweist, und
c. festen Partikeln mindestens eines pharmazeutischen Wirkstoffs (API), wobei die Partikel vorzugsweise einen Durchmesser von ≤ 300 µm aufweisen,
wobei der Silicahydrogel-Verbundstoff bis zu 75 Gewichts-% der nicht aktiven Silica-Partikel umfasst und wobei der Silicahydrogel-Verbundstoff, wenn er ruhend gelagert wird, nicht fließend und strukturell stabil ist, und, wenn durch Injektion Scherspannung angelegt wird, scherverdünnend ist.

2. Silicahydrogel-Verbundstoff nach Anspruch 1, **dadurch gekennzeichnet, dass** das Silicasol einen Feststoffgehalt von 0,5 - 3 Gewichts-%, vorzugsweise 0,5 - 2 Gewichts-%, aufweist.

3. Silicahydrogel-Verbundstoff nach Anspruch 1 oder 2, **dadurch gekennzeichnet, dass** die nicht aktiven Silica-Partikel einen Durchmesser in einem Bereich von 1 µm bis 100 µm, vorzugsweise von 1 µm bis 30 µm, bevorzugter von 1 µm bis 20 µm aufweisen.

4. Silicahydrogel-Verbundstoff nach den Ansprüchen 1, 2 oder 3, **dadurch gekennzeichnet, dass** das Silicasol Silicasolpartikel umfasst, die einen Durchmesser von ≤ 100 nm aufweisen.

5. Silicahydrogel-Verbundstoff nach einem der vorstehenden Ansprüche 1-4, **dadurch gekennzeichnet, dass** der Silicahydrogel-Verbundstoff einen Feststoffgehalt von 20 Gewichts-% bis 80 Gewichts-%, vorzugsweise von 30 Gewichts-% bis 60 Gewichts-%, noch bevorzugter von 35 Gewichts-% bis 55 Gewichts-%, aufweist.

6. Silicahydrogel-Verbundstoff nach einem der vorstehenden Ansprüche 1-5, **dadurch gekennzeichnet, dass** der Silicahydrogel-Verbundstoff weiter aktive Silica-Partikel umfasst, die 0,1 bis 70 Gewichts-%, vorzugsweise 0,3 bis 50 Gewichts-%, und besonders bevorzugt 1 bis 20 Gewichts-% eines pharmazeutischen Wirkstoffs umfassen.

7. Silicahydrogel-Verbundstoff nach einem der vorstehenden Ansprüche 1-6, **dadurch gekennzeichnet, dass** Silica ein von Alkoxysilan abgeleitetes Silica, vorzugsweise ein von Tetraethoxysilan abgeleitetes Silica ist.

8. Silicahydrogel-Verbundstoff nach einem der vorstehenden Ansprüche 1-7, **dadurch gekennzeichnet, dass** die nicht aktiven Silica-Partikel aus der Gruppe ausgewählt sind, bestehend aus sprühgetrockneten Silica-Partikeln; Silica-Faserfragmenten; und geformten oder gegossenen Silica-Monolithen, als solche oder zerkleinert.

9. Silicahydrogel-Verbundstoff nach einem der vorstehenden Ansprüche 1-8, **dadurch gekennzeichnet, dass** die festen Partikel von pharmazeutischen Wirkstoffen aus einer Gruppe ausgewählt sind, bestehend aus Partikeln, die durch Direktsynthese und/oder Ausfällung; Kristallisationsverfahren oder überkritische Fluidtechnologie für kleine Partikel, wie etwa kontrollierte Expansion einer überkritischen Lösung; Auflösungs-Ausfällungs-/Kristallisationszyklus; Sprüh- oder Gefriertrocknung, mechanische Verfahren wie etwa Mahlen, Schlagen und Vermahlen, die verwendet werden, um die Größe von Feststoffen zu verringern, wie etwa Mahlen mit Mörser und Stößel, Nassvermahlen, pneumatisches Vermahlen; Granulierungsverfahren ausgehend von submikronen Partikeln, hergestellt werden.

10. Silicahydrogel-Verbundstoff nach einem der Ansprüche 1-9 zum Verabreichen eines pharmazeutischen Wirkstoffs.

11. Silicahydrogel-Verbundstoff nach Anspruch 10, **dadurch gekennzeichnet, dass** der pharmazeutische Wirkstoff Anagrelid oder eines seiner pharmazeutisch verträglichen Salze ist, das Hydrochloridsalz beinhaltet.

12. Silicahydrogel-Verbundstoff nach Anspruch 10 oder 11, **dadurch gekennzeichnet, dass** die Verabreichung parenteral erfolgt.

13. Silicahydrogel-Verbundstoff nach Anspruch 12, **dadurch gekennzeichnet, dass** die Verabreichung durch Injektion erfolgt.

14. Silicahydrogel-Verbundstoff nach einem der Ansprüche 1-9 zur Verwendung bei der kontrollierten Abgabe eines pharmazeutischen Wirkstoffs.

## Revendications

1. Composite d'hydrogel de silice comprenant au moins un principe pharmaceutique actif, dans lequel le composite d'hydrogel de silice peut être obtenu en mélangeant
a. des particules de silice non actives présentant un diamètre ≤ 100 µm mesuré par diffraction laser,
b. un sol de silice, présentant une teneur en matières solides dans le sol de silice inférieure à 3 % en poids, et
c. des particules solides d'au moins un principe pharmaceutique actif (API), les particules présentant de préférence un diamètre ≤ 300 µm,
dans lequel le composite d'hydrogel de silice comprend jusqu'à 75 % en poids desdites particules de silice non actives et dans lequel le composite d'hydrogel de silice est non fluide et structurellement stable lorsqu'il est stocké au repos et se cisaille lorsque la contrainte de cisaillement est appliquée par injection.

2. Composite d'hydrogel de silice selon la revendication 1, **caractérisé en ce que** le sol de silice présente une teneur en matières solides de 0,5 à 3 % en poids, de préférence 0,5 à 2 % en poids.

3. Composite d'hydrogel de silice selon la revendication 1 ou 2, **caractérisé en ce que** les particules de silice non actives présentent un diamètre dans une plage de 1 µm à 100 µm, de préférence de 1 µm à 30 µm, plus préférentiellement de 1 µm à 20 µm.

4. Composite d'hydrogel de silice selon les revendications 1, 2 ou 3, **caractérisé en ce que** le sol de silice comprend des particules de sol de silice présentant un diamètre ≤ 100 nm.

5. Composite d'hydrogel de silice selon l'une quelconque des revendications 1-4 précédentes, **caractérisé en ce que** le composite d'hydrogel de silice présente une teneur en matières solides de 20 % en poids à 80 % en poids, de préférence de 30 % en poids à 60 % en poids, encore plus préférentiellement de 35 % en poids à 55 % en poids.

6. Composite d'hydrogel de silice selon l'une quelconque des revendications 1-5 précédentes, **caractérisé en ce que** le composite d'hydrogel de silice comprend en outre des particules de silice actives comprenant de 0,1 à 70 % en poids, de préférence de 0,3 à 50 % en poids, et le plus préférentiellement de 1 à 20 % en poids d'un agent pharmaceutique actif.

7. Composite d'hydrogel de silice selon l'une quelconque des revendications 1-6 précédentes, **caractérisé en ce que** la silice est une silice dérivée d'alcoxysilane, de préférence une silice dérivée de tétraéthoxysilane.

8. Composite d'hydrogel de silice selon l'une quelconque des revendications 1-7 précédentes, **caractérisé en ce que** les particules de silice non actives sont choisies dans le groupe consistant en des particules de silice séchées par pulvérisation ; des fragments de fibres de silice ; et des monolithes de silice moulés ou coulés, tels quels ou broyés.

9. Composite d'hydrogel de silice selon l'une quelconque des revendications 1-8 précédentes, **caractérisé en ce que** les particules solides de principes pharmaceutiques actifs sont choisies dans un groupe consistant en des particules préparées par synthèse directe et/ou précipitation ; des procédés de cristallisation ou une technologie des fluides supercritiques pour les petites particules, telles que l'expansion contrôlée d'une solution supercritique ; un cycle de dissolution- précipitation/cristallisation ; des procédés mécaniques de séchage par pulvérisation ou lyophilisation, tels que le broyage, le concassage et le fraisage, utilisés pour réduire la taille des matières solides, tels que le broyage au mortier et au pilon, le broyage humide, le broyage pneumatique ; des procédés de granulation à partir de particules submicroniques.

10. Composite d'hydrogel de silice selon l'une quelconque des revendications 1-9 pour l'administration d'un principe pharmaceutique actif.

11. Composite d'hydrogel de silice selon la revendication 10, **caractérisé en ce que** le principe pharmaceutique actif est l'anagrélide ou l'un quelconque de ses sels pharmaceutiquement acceptés, incluant le sel de chlorhydrate.

12. Composite d'hydrogel de silice selon la revendication 10 ou 11, **caractérisé en ce que** l'administration est parentérale.

13. Composite d'hydrogel de silice selon la revendication 12, **caractérisé en ce que** l'administration se fait par injection.

14. Composite d'hydrogel de silice selon l'une quelconque des revendications 1-9 destiné à être utilisé dans la délivrance contrôlée d'un principe pharmaceutique actif.
